Europäisches Patentamt

European Patent Office

Office européen des brevets

(11) Publication number: **0 224 981**
**A2**

(12) # EUROPEAN PATENT APPLICATION

(21) Application number: 86305511.7

(51) Int. Cl.⁴: **A61L 15/03** , **A61K 47/00**

(22) Date of filing: 17.07.86

A request for correction of a number of terms in the description has been filed pursuant to Rule 88 EPC. A decision on the request will be taken during the proceedings before the Examining Division (Guidelines for Examination in the EPO, A-V, 2.2).

(30) Priority: 04.11.85 US 795047

(43) Date of publication of application:
10.06.87 Bulletin 87/24

(84) Designated Contracting States:
BE DE FR GB IT LU NL SE

(71) Applicant: PACO RESEARCH CORPORATION
1705 Oak Street
Lakewood New Jersey 08701(US)

(72) Inventor: Kim, K. Benjamin
226 Middle Drive
Toms River New Jersey 08753(US)

(74) Representative: Oliver, Roy Edward et al
POLLAK MERCER & TENCH High Holborn
House 52-54 High Holborn
London WC1V 6RY(GB)

(54) Nitroglycerin transdermal delivery system.

(57) A transdermal delivery system for a vasoactive substance includes, by weight, about 30 to 70% of a medically-acceptable liquid silicone rubber, about 5 to 25% of a vasoactive substance, about 0.1 to 2.0% of a gelling agent, about 2 to 15% of a material which swells as water is absorbed from the stratum corneum and about 5 to 35% of a mixture comprising one or more solvents, viscosity reducers and skin penetration enhancers including, by weight of the entire delivery system, about 5 to 25% of a solvent, about 0 to 5% of a viscosity reducer and about 5 to 25% of a skin penetration enhancer.

EP 0 224 981 A2

## NITROGLYCERIN TRANSDERMAL DELIVERY SYSTEM

Scope Of The Invention

The present invention is directed to a nitroglycerin transdermal delivery system. This delivery system dispenses a substantially uniform flux of nitroglycerin.

Background Of The Invention

Nitrogylcerin, a vasoactive substance, has been used in the treatment of angina for more than 100 years. Originally, it was taken internally and its effects were noticeable within about 2 minutes. To sustain the pharmaceutical effect, doses were administered every 2 hours.

Recently transdermal delivery systems have become popular. These systems allow a controlled amount of nitrogylcerin to be continuously administered over a sustained period of time. Examples of these systems are found in U.S. Patent Nos. 4,486,193; 4,336,243; 4,291,015; and 3,742,951.

The release rate of vasoactive substances from the prior art delivery systems is controlled by the mass transfer of the substance through a matrix or diffusion membrane. As the concentration of the substance decreases, the rate of diffusion of the vasoactive substance also decreases. Thus, the transfer of the substance into the patient may be continuous but it is not uniform.

Summary Of The Invention

The present invention relates to a transdermal delivery system for the administration of vasoactive substances, particularly nitrogylcerin. The described invention includes a patch comprising a polymer matrix which is applied to the skin. A vasoactive substance which is dispersed throughout the polymeric matrix can diffuse from the matrix and transdermally enter the human body. The flux of nitroglycerin is substantially uniform.

The transdermal delivery system for the vasoactive substance includes on a weight percentage basis of the entire delivery system from about 30 to 70% of a medically acceptable silicone rubber, from about 5 to 25% of a vasoactive substance, from about 0.1 to 2% of a gelling agent, from about 2 to 15% of a material which swells as water is absorbed from the stratum corneum, and from about 5 to 35% of a mixture of solvents, viscosity reducers and skin penetration enhancers.

The delivery system is formed into a thin patch which is sealed into an impervious container until use. When used a single face of the patch is exposed and placed in contact with the stratum corneum.

Description Of The Drawings

For the purpose of illustrating the invention, there is shown in the drawing a graphical representation of experimental results obtained from various specific embodiments which are set forth below in the examples; it being understood, however, that this invention is not limited to the precise mixtures and specific constituents shown or described.

The Figure is a graph illustrating the substantially uniform flux of nitroglycerin obtained from the examples of the delivery system.

Detailed Description Of The Invention

In the present invention, the transdermal delivery system comprises a polymeric diffusion matrix which is formed into thin patches and sealed within an impervious container until use. The matrix composed on a weight percentage basis of the entire delivery system from about 30 to 70% of a medically acceptable liquid silicone rubber, from about 5 to 25% of the vasoactive substance, from about 0.1 to 2.0% of a gelling agent, from about 2 to 15% of a material which swells as water is absorbed from the stratum corneum; and from about 5 to 35% of a mixture of a solvent, viscosity reducer and skin penetration enhancer. The mixture further includes from about 5 to 25% of a solvent, from about 0 to 5% of a viscosity reducer and from about 5 to 25% of a skin penetration enhancer.

A preferred medically acceptable liquid silicon rubber is silastic Q7 4840A/B medical grade liquid silicon rubber manufactured by Dow Corning Corporation of Midland, Michigan. This rubber is a two component system which must be mixed prior to being cross-linked. Also preferred are any biologically acceptable polymer rubbers which are capable of forming thin walls or coatings through which pharmaceuticals can pass at a controlled rate.

The vasoactive substance is preferably a nitrogylcerin based medication such as 10% nitrogylcerin on lactose. Additionally, isosorbide dinitrate is contemplated as a candidate for use in the present invention.

The gelling agent is preferably colloidal silicon dioxide. The colloidal silicon dioxide absorbs a great amount of liquid and becomes a gel.

The material which swells as water is absorbed from the stratum corneum is cellulose and its derivatives or starch and its derivatives. These materials are added to the matrix to facilitate the release of the vasoactive substance. As these materials absorb water from the stratum corneum, they swell. The matrix is thusly expanded, thereby facilitating the controlled release and the substantially uniform flux of the vasoactive substance obtainable from the present invention. The cellulose and cellulose derivatives include microcrystalline cellulose, methyl cellulose, sodium methyl cellulose, ethyl cellulose or mixtures thereof. The starch and starch derivatives include corn starch, wheat starch, rice starch, soluble starch, plant root starch, potato starch, sodium starch glycolate or mixtures thereof. Preferably sodium starch glycolate which is sold under the tradename PRIMOJEL manufactured by Generichem Corp. of Little Falls, New Jersey. Also preferred is a microcrystalline cellulose sold under the tradename AVICEL by the FMC Co. of Philadelphia, Pennsylvania.

The solvent used in the preferred embodiment of the present invention is isopropyl mylistate. The solvents used to dissolve the vasoactive substance and aid in the dispersement of the vasoactive substance throughout the delivery system. Other solvents such as isopropyl oleate, isopropyl stearate, isopropyl lurate, isopropyl palmitate, isopropyl linoleate, isopropyl sorbate, glycerol, $C12$ -$C15$ alcohol benzoate and mixtures thereof can be used.

The viscosity reducing agent is preferably silicon oil. The preferred silicon oil is Dow Corning medial fluid 360 manufactured by Dow-Corning Corporation of Midland, Michigan. The viscosity reducing agent is used to reduce the visocity of the rubber. Other viscosity reducing agents such as mineral oil, polymethyl siloxane or mixtures thereof may also be used.

The penetration enhancers may include decyl alcohol, undecyl alcohol, dodecyl alcohol propylene glycol, polyethylene glycol, $C9$ -$C11$, $C12$ -$C13$ and $C12$ -$C15$ fatty alcohols or mixtures thereof The penetration enhancers facilitate the transport of the vasoactive substance through the patients' skin.

The liquid silicon rubber is cross-linked in situ. The cross-linking of the rubber is performed after the formulation containing the other constituents are added to the rubber. When the nitrogylcerin patch is applied on the skin, the nitrogylcerin diffuses through the delivery system at a therapeutically effective constant rate. The cellulose, cellulose derivatives, starch and starch derivatives absorb moisture from the stratum corneum which contains approximately 20% water and gradually expands in volume thus allowing a continual and substantially uniform release of nitrogylcerin.

The preparation of the transdermal delivery system according to the present invention is accomplished as follows. The two components of the liquid silicon rubber are mixed with approximately 0 to 5% of silicon oil to reduce the viscosity of the rubber mixture. The vasoactive substance is mixed with the solvent and the penetration enhancer. Thus, the vasoactive substance is dissolved into the mixture of solvent and penetration enhancer. The material which swells and the gelling agent are then added to the mixture of nitrogylcerin, solvent and penetration enhancer. This entire mixture is then added to the rubber mixture. The entire delivery system formulation is mixed for about 10 to 20 minutes in a low shear, explosion proof mixing vessel maintained under vacuum of about 50 to 75 cm Hg.

An other preparation of this invention include mixing a first component of the liquid silicon rubber with the mixture of the vasoactive substance, solvent and penetration enhancer, the material which swells and the gelling agent for about 5 to 15 minutes in a low shear, explosion proof mixing vessel under vacuum. Then the second component of the liquid silicon rubber is added and mixed under vacuum for about 15 to 25 minutes. Furthermore, other mixing methods are disclosed in the examples below.

3

The product of any of the disclosed mixing methods is transferred to an aluminum foil lined stainless steel concave plate. The depth of the cavity formed in the concave plate is approximately 0.2 to 4 centimeters. A metallized polyethylene film or aluminum foil is placed on top of the mixture. A top plate is placed on top of the polyethylene film or aluminum foil. The concave plates and top plate are secured in place with a screw located in each of the four corners. The mold is placed in an oven which circulates air at approximately 60° centigrade. Alternatively a mold can be formed using a suitably sized preform aluminum cup having an aluminum lid. The cup is filled with the formulation. The lid is placed over the cup and sealed. The filled cup and lid are placed in an oven which circulates air at about 60° centigrade. The curing time for the formulation is approximately 2 to 3 hours. The cured matrix is then cut into disks having approximately a 10 cm² surface area. The disks are transferred to preformed aluminum cups and sealed. The size of the disk is determined according to the nitrogylcerin content in the matrix and the optimum dosage is determined from a series of bioavailability tests.

The invention is further illustrated but is not intended to be limited by the following examples in which all parts and percentages are by weight unless otherwise specified.

Example 1

A 10 percent nitrogylcerin on lactose (25g) are mixed with isopropyl mylistate (5g) and dodecyl alcohol (23g) for 5 minutes. The nitrogylcerin solution is added to the liquid silicon rubber (Dow Corning Q7-4840 A/B, 15g of each component). Colloidal silicon dioxide (2g) and sodium starch glycolate (15g PRIMOJEL) are mixed into the rubber, nitroglycerin solution for about 10 minutes and then deaerated in a vacuum chamber for about 10 minutes. A uniform paste is obtained. This paste is transferred into the aluminum foil lined mold plate and covered with an aluminum foil. A stainless steel top is placed on the covered aluminum foil and secured with a screw located in each of the four corners. This mold is placed in an air-circulating oven set at about 60°C for approximately two and one-half hours. After cooling, the cured matrix is removed from the mold and cut into 3.3 x 3.3 cm patches and put into aluminum pouches and sealed.

Example 2

Liquid silicon rubber (Dow Corning Q7-4840) ( 35g of each component) are mixed with silicon oil (5g) for 5 minutes. This liquid rubber mixture is deaerated under the vacuum chamber for about 10 minutes. A 10 percent nitrogylcerin on lactose (5g) is mixed with propylene gylcol (5g) and isopropyl mylistate (12.9g) for about 5 minutes. This nitrogylcerin solution, sodium starch gylcolate (2g PRIMOJEL) and colliodal silicon dioxide (9.1g) are added to the above liquid silicon rubber mixture. The mixture is blended for about 10 minutes and deaerated in a vacuum chamber. The final steps are the same as in Example 1.

Example 3

A 10 percent nitrogylcerin on lactose (11 grams) is mixed with isopropyl mylistate (15g) and dodecyl alcohol (6g) for about 5 minutes. Liquid silicon rubber (27 grams of each component) is mixed with the sodium starch gylcolate (7g PRIMOJEL) for about 5 minutes. The nitrogylcerin solution and colloidal silicon dioxide (1g) are added to the liquid silicon rubber mixture and mixed for about 15 minutes. This well mixed paste is deaerated in a vacuum chamber for about 10 minutes. The final steps are the same as in Example 1.

Example 4

Liquid silicon rubber (24.5 grams of each component) are mixed with sodium starch gylcolate (4.5g PRIMOJEL) for about 5 minutes. A 10 percent nitrogylcerin on lactose (10g) is mixed with isoproyl mylistate (23g) and dodecyl alcohol (12g) for about 5 minutes. This nitrogylcerin mixture and colloidal silicon dioxide (1.5g) are added to the liquid silicon rubber mixture and blended for about 15 minutes. This uniformed paste is deaerated in a vacuum chamber for about 10 minutes.

4

Example 5

Following the preparation method of Example 1, nitroglycerin patches are prepared from 10 percent nitroglycerin on lactose (15g), starch (12g), isopropyl mylistate (17g), colloidal silicon dioxide (2g), dodecyl alcohol (15g), and liquid silicon rubber mixture (40g). The patches are put into aluminum pouches and sealed.

Example 6

Following the preparation method of Example 1, nitrogylcerin patches are prepared from 10 percent nitro gylcerin on lactose (14g), starch (7g), silicon oil (5g), isopropyl mylistate (17g), colloidal silicon dioxide (2g), dodecyl alcohol (15g) and liquid silicon rubber mixture (40g). The patches are kept in aluminum pouches until use.

Example 7

Following the preparation method of Example 1, nitroglycerin patches are prepared from 10 percent nitrogylcerin on lactose (14g), sodium starch glycolate (15g), silicon oil (5g), colloidal silicon dioxide (1.5g), isopropyl mylistate (13.25g), dodecyl alcohol (11.25g), and liquid silicon rubber mixture (40g). The patches are kept in aluminum pouches until use.

Example 8

Following the preparation method of Example 1, nitroglycerin patches are prepared from 10 percent nitrogylcerin on lactose (7g), silicon oil (2.5g), starch (5.9g), isopropyl mylistate (6.625g), colloidal silicon dioxide (0.759g), dodecyl alcohol (5.625g), and liquid silicon rubber mixture (22.5g). The patches are kept in aluminum pouches until use.

Example 9

Following the preparation method of Example 1, nitroglycerin patches are prepared from 10 percent nitrogylcerin on lactose (7g), propylene glycol (2.5g), colloidal silicon dioxide (0.75g), silicon oil (2.5g), isopropyl mylistate (9.75g), sodium starch glycolate (5.0g) and liquid silicon rubber mixture (22.5g). The patches are kept in aluminum pouches until use.

Example 10

Following the preparation method of Example 1, nitroglycerin patches are prepared from liquid silicon rubber mixture (22.5g), silicon oil (2.5g), sodium starch glycolate (5g), colloidal silicon dioxide (0.75g), isopropyl mylistate (6.625g), undecyl alcohol (5.625g), and 10 percent nitroglycerin on lactose (7g). The patches are kept in aluminum pouches until use.

Example 11

To evaluate the nitroglycerin release rate of each formulation in vitro hairless mouse Franz Cell Assays are conducted. The membranes used for the percutaneous absorption studies are full thickness skins taken from the abdominal surface of the hairless mouse SKH-HR strain. Hairless mice (males), 8-10 weeks old, are sacrificed by snapping the spinal cord at the neck. Two rectangular sections of abdominal skin, several centimeters in each dimension are excessed from the animal with surgical scissors.

After the excised skin is trimmed into an oversized, rough circle, it is mounted between the two compartments of a diffusion cell. the receptor compartment is then filled with normal saline to keep the membrane moist. The saline is equilibrated to 37°C by circulating water through an outer jacket. After an equilibration period of 1 hour, the receptor compartment is evacuated completely and refilled with fresh normal saline preequilibrated to the experimental temperature (37°C). At this point, the donor compartment is charged with the test formulation. The amount of formulation applied should be just enough to cover the exposed area of the skin. The length of the experimental run should be adjusted such that not more than 10 percent of the applied amount permeates the skin. It is advisable to empty the receptor solution at each sampling time interval and refill with fresh normal saline preequilibrated to the experimental temperature. For adjustment of sampling time intervals, an ideal consideration would be to have the concentration of the active ingredient in the receptor solution at any time course of the experimental run never to exceed 10 percent of the solubility value.

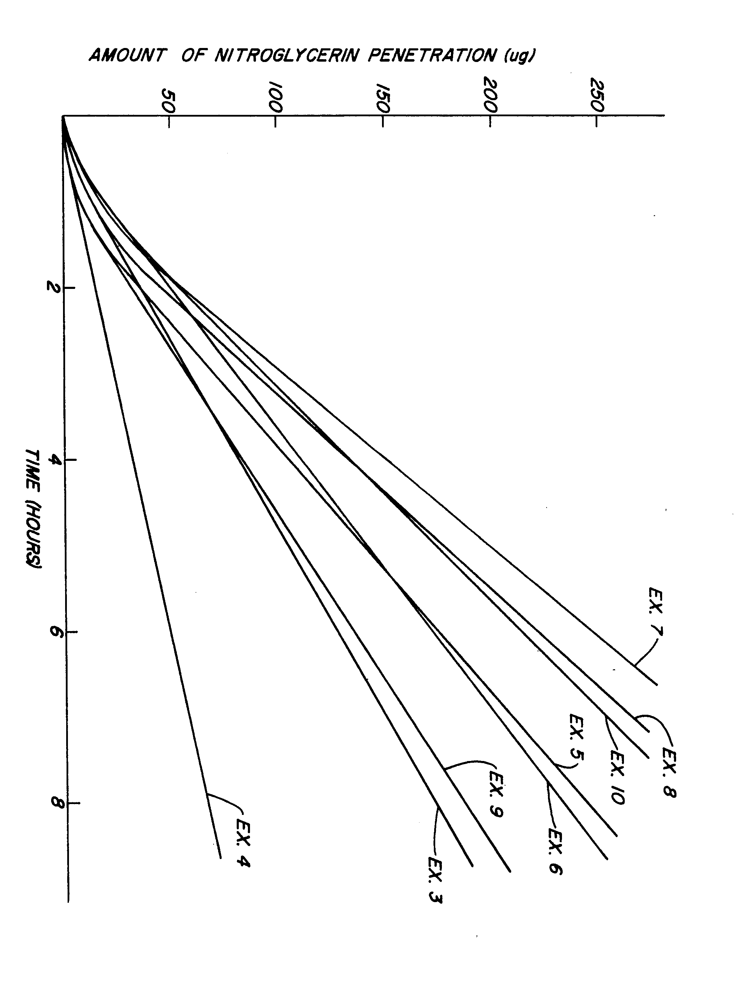

The results of Franz Cell Assay for Examples 3-10 are tabularly presented in Table I and graphically illusrated in Figure 1. As can be appreciated from the Table and Figure the flux of nitroglycerin is substantially uniform.

TABLE I

| Samples | | Hours | Amount of Penetration (ug) | Total Amount (ug) | Penetration (ug/hr) | Flux ug/hr-cm$^2$ | Average Flux |
|---|---|---|---|---|---|---|---|
| | A | 2 | 35.286 | 35.286 | s = 21.376 | | |
| | | 4 | 42.487 | 77.773 | t = 0.3534 | 27.231 | |
| 3 | | 6 | 43.017 | 120.790 | r = 1.0000 | | 27.244 |
| | B | 2 | 35.205 | 35.205 | s = 21.396 | 27.256 | |
| | | 4 | 46.547 | 81.752 | t = 0.2961 | | |
| | | 6 | 51.474 | 133.226 | r = 0.9987 | | |
| | A | 2 | 17.419 | 17.419 | s = 10.101 | 12.983 | |
| | | 4 | 22.657 | 40.076 | t = 0.2165 | | 10.962 |
| 4 | | 6 | 18.110 | 58.186 | r = 0.9979 | | |
| | B | 2 | 9.563 | 9.563 | s = 7.018 | 8.940 | |
| | | 4 | 14.556 | 24.119 | t = 0.6126 | | |
| | | 6 | 13.515 | 37.634 | r = 0.9998 | | |
| | A | 2 | 28.301 | 28.301 | s = 27.673 | 35.252 | |
| | | 4 | 49.968 | 78.269 | t = 1.0421 | | |
| 5 | | 6 | 60.725 | 138.994 | r = 0.9984 | | 43.918 |
| | B | 2 | 46.532 | 46.532 | s = 41.278 | 52.583 | |
| | | 4 | 77.372 | 123.904 | t = 0.9146 | | |
| | | 6 | 87.740 | 211.644 | r = 0.9993 | | |
| | A | 2 | 33.023 | 33.023 | s = 22.083 | | |
| | | 4 | 40.689 | 73.712 | t = 0.5571 | 28.131 | |
| 6 | | 6 | 47.642 | 121.354 | r = 0.9990 | | 40.133 |
| | B | 2 | 63.733 | 63.733 | s = 40.925 | | |
| | | 4 | 81.215 | 144.948 | t = 0.4478 | 52.134 | |
| | | 6 | 82.484 | 227.432 | r = 1.0000 | | |

7

TABLE I

| Samples | Hours | Amount of Penetration (ug) | Total Amount (ug) | Penetration (ug/hr) | Flux ug/hr-cm$^2$ | Average Flux |
|---|---|---|---|---|---|---|
| A | 2 | 43.524 | 43.524 | s = 39.457 | 50.264 | |
| | 4 | 78.246 | 121.770 | t = 0.9026 | | 59.424 |
| 7 | 6 | 79.582 | 201.352 | r = 1.0000 | | |
| B | 2 | 71.634 | 71.634 | s = 53.838 | | |
| | 4 | 103.833 | 180.467 | t = 0.6623 | 68.583 | |
| | 6 | 106.519 | 286.986 | r = 1.0000 | | |
| A | 2 | 45.215 | 45.215 | s = 42.288 | 53.870 | |
| | 4 | 82.394 | 127.609 | t = 0.9480 | | 54.930 |
| 8 | 6 | 86.759 | 214.368 | r = 0.9999 | | |
| B | 2 | 48.227 | 48.227 | s = 43.951 | 55.989 | |
| | 4 | 84.157 | 132.384 | t = 0.9311 | | |
| | 6 | 91.645 | 224.029 | r = 0.9997 | | |
| A | 2 | 34.613 | 34.613 | s = 27.456 | 34.876 | |
| | 4 | 55.253 | 89.866 | t = 0.7352 | | |
| 9 | 6 | 54.569 | 144.435 | r = 1.0000 | | 32.636 |
| B | 2 | 30.863 | 30.863 | s = 23.861 | 30.396 | |
| | 4 | 48.842 | 79.405 | t = 0.6951 | | |
| | 6 | 46.900 | 126.305 | r = 1.0000 | | |
| A | 2 | 53.344 | 53.344 | s = 40.014 | 50.973 | |
| | 4 | 77.202 | 130.546 | t = 0.6904 | | |
| 10 | 6 | 82.852 | 213.398 | r = 0.9998 | | 50.212 |
| B | 2 | 56.148 | 56.148 | s = 38.819 | 49.451 | |
| | 4 | 82.054 | 138.202 | t = 0.5157 | | |
| | 6 | 73.222 | 211.424 | r = 0.9995 | | |

s = Slope

t = Lag Time

r = Correlation Coefficient

It is to be understood that any of the components and conditions mentioned as suitable herein can be substituted for its counterpart in the foregoing examples and that although he invention has been described in considerable detail in the foregoing, such detail is solely for the purpose of illustration. Variations can be made in the invention by those skilled in the art without departing from the spirit and scope of the invention except as set forth in the claims.

**Claims**

1. A transdermal delivery system for a vasoactive substance, characterised by comprising:

about 30 to 70% by weight of a medically-acceptable liquid silicone rubber;

about 5 to 25% by weight of a vasoactive substance;

about 0.1 to 2.0% by weight of a gelling agent;

about 2 to 15% by weight of a material which swells as water is absorbed from the stratum corneum; and about 5 to 35% by weight of a mixture comprising one or more solvents, viscosity reducers and/or skin penetration enhancers.

2. A system according to claim 1, wherein the mixture includes:

about 5 to 25%, by weight of the entire delivery system, of a solvent;

about 0 to 5%, by weight of the entire delivery system, of a viscosity reducer; and about 5 to 25%, by weight of the entire delivery system, of a skin penetration enhancer.

3. A system according to claim 1 or 2, wherein the vasoactive substance is 10% nitroglycerin or lactose.

4. A system according to claim 1, 2 or 3, wherein the gelling agent is colloidal silicon dioxide.

5. A system according to any preceding claim, wherein the material which swells is selected from cellulose, microcrystalline cellulose, methyl cellulose, sodium methyl cellulose and ethyl cellulose.

6. A system according to any preceding claims, wherein the material which swells is selected from corn starch, wheat starch, rice starch, soluble starch, plant root starch, potato starch and sodium starch glycolate.

7. A system according to any preceding claim, wherein the solvent is selected from isopropyl mylistate, isopropyl palmitate, isopropyl stearate, isopropyl oleate, isopropyl linoleate, isopropyl sorbate, isopropyl lurate, glycerol and C12 -C15 alcohol benzoate.

8. A system according to any preceding claim, wherein the viscosity reducer is selected from silicone oil, mineral oil and polymethyl siloxane.

9. A system according to any preceding claim, wherein the penetration enhancer is selected from decyl alcohol, undecyl alcohol, dodecyl alcohol, propylene glycol, polyethylene glycol and C9 -C11, C12 -C13 and C12 -C15 fatty alcohols.

10. A system according to any preceding claim, which is formed into a thin patch having a surface area of approximately 10 cm$^2$ and a depth of about 0.2 to 4.0 cm.